(19) Europäisches Patentamt
European Patent Office
Office européen des brevets

(11) **EP 3 943 918 A1**

(12) **EUROPEAN PATENT APPLICATION**

(43) Date of publication:
**26.01.2022 Bulletin 2022/04**

(21) Application number: **21187313.8**

(22) Date of filing: **22.07.2021**

(51) International Patent Classification (IPC):
**G01N 21/53** (2006.01)     **G01N 21/63** (2006.01)
**G01N 21/64** (2006.01)     **G01N 21/49** (2006.01)
**B01L 3/00** (2006.01)       **G01N 33/18** (2006.01)

(52) Cooperative Patent Classification (CPC):
**G01N 21/6486; B01L 3/502715; G01N 15/1031;**
**G01N 15/1436; G01N 15/1484; G01N 21/53;**
**G01N 21/645; G01N 33/1826;** B01L 2200/027;
B01L 2300/0654; B01L 2300/0825;
G01N 2015/0065; G01N 2015/0073;
G01N 2015/1006; G01N 2015/1486;          (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **22.07.2020  PT 2020116598**

(71) Applicant: **Universidade do Minho**
**4704-553 Braga (PT)**

(72) Inventors:
• **VALENTE GONÇALVES, Luís Miguel**
**4715-404 Braga (PT)**

• **HENRIQUES MINAS, Graça Maria**
**4710-588 Adaúfe (PT)**
• **GONÇALVES PINTO, Vânia Cristina**
**4705-769 Braga (PT)**
• **TEIXEIRA DE SOUSA, Paulo Jorge**
**4705-769 Braga (PT)**
• **MOTA CARVALHO, Denise Andreia**
**5050-030 Fontelas (PT)**
• **FERNÁNDEZ SUÁREZ, Emilio**
**36310 Vigo (PT)**

(74) Representative: **Patentree**
**Edificio Net**
**Rua de Salazares, 842**
**4149-002 Porto (PT)**

(54) **DEVICE FOR IDENTIFICATION AND QUANTIFICATION OF PHYTOPLANKTON, METHODS AND USES THEREOF**

(57)     The present disclosure is related to a lab-on-a-chip device and method for *in-situ* and *in-vivo* identification and quantification of phytoplankton cell comprising: an inlet and an outlet; a constriction microchannel that allows the passage of one single cell through the microchannel; and at least four measurement lines configured to identify and quantify a pre-defined phytoplankton cell, wherein each line comprises: one light source, preferably LEDs or laser; at least three photodiodes; wherein at least 2 photodiodes are different and wherein the three photodiodes are selected from a fluorescence photodiode, scattering photodiode, and transmission photodiode; and at least one optical filter, wherein the optical filter is aligned with the fluorescent photodiode; wherein the scattering photodiode, and fluorescence photodiode are not aligned with the source light.

EP 3 943 918 A1

(52) Cooperative Patent Classification (CPC): (Cont.)
G01N 2015/1493; G01N 2015/1497;
G01N 2021/635; G01N 2021/6482

**Description**

**TECHNICAL FIELD**

The present disclosure relates to a device used for identification and *in-situ* and *in-vivo* quantification of phytoplankton cells, comprising a lab-on-a-chip with a constriction channel, where the cells are forced to pass through 4 to 12 optical measurement lines.

**BACKGROUND**

The sample collection and laboratory analysis of phytoplankton species and their abundance is a routine task in marine scientific research and local authorities. Certain phytoplankton species produce biotoxins responsible for the so-called "red tides" or for the proliferation of harmful algae, which are capable of killing marine life, as well as the people who feed on them [1-4]. Therefore, it is of extreme importance to study the population dynamics of phytoplankton communities enabling a knowledge about the potentially harmful algae in aquatic systems [5]. This task is performed by local authorities, that routinely collect phytoplankton and shellfish samples, and then analyse their toxicity at laboratory, and regulate its harvesting, promoting seafood quality and safety [5].

In phytoplankton, there are several photosynthetic pigments, such as chlorophylls a, b and c, carotenes, xanthophylls and phycobilins, being chlorophyll the main photosynthetic pigment of all organisms. Measuring photosynthetic phytoplankton pigments can help the identification of phytoplankton species [6, 7].

Several techniques, as well as commercial equipment are used to detect, quantify and classify the phytoplankton [8, 9]. Most of them use the phytoplankton optical characteristics, such as, their natural absorption of light in the visible spectral range and their natural fluorescence in the 600 nm to 750 nm spectral range. However, mainly due to the low directivity and selectivity of equipment's, analysis is always performed with a previous laboratory procedure, to extract pigments from algae using solvents, technique that is not suitable for in-situ and in-vivo analysis.

A chlorophyll sensor probe, capable of measuring chlorophyll and phytoplankton mass was described in the patent document KR20160089967 (A). The described device is based on the fluorescence measurement of chlorophyll a and b of the phytoplankton, using two excitation LEDs in wavelength range of 430-480 nm, and a single detector for the range 600-700nm. This device is proposed to measure chlorophyll and phytoplankton mass, but identification of algae species is not described in this device.

The patent document CN105628657 (A) describes a device for quantifying chlorophyll by fluorescence that can be used for *in situ* water eutrophication detection and monitoring of lakes, rivers and the sea. It is disclosed its high sensitivity and speed, not being necessary to use reagents for the detection, using 1024Hz pulsed excitation at 460 nm and detection with a photodiode. The device makes analyses in large volume of water, with many phytoplankton species. An average measure is obtained considering all different cells in the volume. For this reason, is unable to identify and quantify the different cells in the water sample analysed.

The patent document CN105136763 (A) discloses a single microalgae cell monitoring device and method based on gas-liquid interface single cell capture system. Cells are captured in bubbles, generated in the transport liquid, and the single cells analysed by chlorophyll fluorescence characterization, using laser excitation and a photoelectric tube detector. High accuracy is obtained, since single cells are analysed, eliminating the need of statistical averages and blur effect, inherent to systems with larger sampling volumes and many cells analysed at same time. However, the emission and detection system used do not allow any cell identification.

The patent document CN103868901 (A) discloses a class phytoplankton identification device and method based on discrete three-dimensional fluorescence spectrum. The device comprises a multiband light-emitting diode (LED) array (excitation light) and a multiband light filter group as a band selector for receiving fluorescence at several wavelengths for obtain the fluorescence spectral characteristics of different classes of phytoplankton in conjunction with phytoplankton pigment composition. The device is compact and is suitable for rapid identification and measurement of field phytoplankton. However, the device does not allow single microalgae cell identification and could be affected by blur effect, inherent to systems with larger sampling volumes and many cells analysed at same time.

The patent document CN104101588 (A) discloses an integrated seawater chlorophyll microfluidic chip sensor, capable of enhancing the anti-interference ability and of improving the fluorescence detection efficiency. The invention also discloses a method for the device manufacturing that includes LEDs, detectors, microfluidic channels and mirrors in the same device. Up to five different wavelengths may be disposed. Despite using lab-on-a-chip technology, it doesn't perform any cell sorting function, able to perform single cell analysis. The device makes analyses in a volume of water with many cells, with many phytoplankton species, and an average measure is obtained considering all different cells in the volume. For this reason, it is unable to identify and quantify the different cells present in the water sample analysed.

The patent document CN105548128 (A) discloses a method and device for detecting the chlorophyll concentration of coastal water body, *in situ,* through a double optical path method, where a beam splitter divides excitation light (blue LED) between sample and a reference photodiode, thus compensating light fluctuations. According to the method, the concentration of the chlorophyll a is detected by directly measuring the fluorescence intensity, compensated with reference measurements. The device is of low size, low weight, low-cost, easy to operate, featuring high accuracy and sensitivity and capable of being applied to multiple detection fields. The invention proposes the measurement of chlorophyll, but it is silent regarding identification of species.

The feasibility of using fluorescence excitation-emission matrices was described [10] for qualitative and quantitative discrimination of five algal taxonomic groups. The results indicate that the fluorometric technique could differentiate algal taxonomic groups accurately at the division level. However, the pigments were previously extracted with a solvent (N, N-dimethylformamide) before fluorescence analysis. Despite promising technique, the analysis was not performed in *in-vivo* samples, but with pigments extracted with a solvent. Therefore, it is not adequate for in *in-vivo* samples.

The feasibility of using fluorescence excitation-emission matrix along with parallel factor analysis and nonnegative least squares method for the differentiation of phytoplankton taxonomic groups was investigated in [10]. Samples collected from the Changjiang River estuary were analysed and five phytoplankton groups were differentiated (Bacillariophyta, Chlorophyta, Dinophyta, Cryptophyta, and Cyanophyta), from the list of 41 phytoplankton species studied. The differentiation results by the proposed fluorometric technique were in good agreement with those from HPLC-CHEMTAX analysis. The results indicate that the fluorometric technique could differentiate algal taxonomic groups accurately at the division level. However, the pigments were extracted from the filters in 10 mL extraction solvent (N, N-dimethylformamide: DMF), which makes this technique unsuitable for *in-situ* analyses.

Allison Schaap, et al.,"Optical classification of algae species with a glass lab-on-a-chip" [16] discloses a method for measuring the intensity distribution of the light with an off-chip four quadrant detector. This method only analyses the size of the algae cells and the distribution depends of the size of algae cells. This method is not able to differentiate phytoplankton cells that have similar size and the results can be ambiguous.

The document CN106872424 discloses an in-situ online detection device for phytoplankton based on photosynthetic pigment fluorescence. The in-situ online detection device comprises an LED (Light Emitting Diode) annular light source, a cylindrical water sample pool, a fluorescent detection module, a microcontrol circuit module, a casing and a bottom cover. However, this device only reflects the overall biomass of phytoplankton, becoming impossible to distinguish and identify individual cells. This device only analyse photosynthetic pigments, does not allow single microalgae cell identification and could be affected by blur effect, inherent to systems with larger sampling volumes and many cells analysed at same time.

None of the previous disclosures presents an accurate device for phytoplankton identification that analyse individual cells, which can provide more sensitivity for early detection of harmful algal blooms (HABs).

These facts are disclosed in order to illustrate the technical problem addressed by the present disclosure and limitations of previous addressed proposals.

## GENERAL DESCRIPTION

The present disclosure relates to a device for *in-situ* and *in-vivo* identification and quantification of phytoplankton cells, based on the optical properties of each cell.

The present disclosure relates to an accurate lab-on-a-chip device for simultaneous characterization of cell size and/or shape, light transmission and specific photosynthetic pigments composition of each specie which provide relevant information for phytoplankton species identification. By analysing individual cells, provides more sensitivity for early detection of harmful algal blooms (HABs).

With the present device it is also possible to differentiate different species that have similar size.

The lab-on-a-chip device may comprises an inlet and an outlet; a constriction microchannel that allows the passage of one single cell through the microchannel; at least four measurement lines configured to identify and quantify a pre-defined phytoplankton cell, wherein each line comprises one light source, preferably LEDs or lasers; at least three photodiodes; and at least one optical filter, wherein the optical filter is aligned with a photodiode. Preferably, each measurement line includes a light emitting diode (LED) or laser, and three photodiodes for fluorescence, scattering and transmission light analysis, respectively. Each measurement line can excite the cell in a single wavelength and analyse its optical transmission, scattering and fluorescence. The device can have from 4 to 12 of these measurement lines.

In this disclosure, scattering photodiode is the same as a dispersion photodiode.

In an embodiment, the use of a small spot analysis, focusing the LED or laser light in each phytoplankton cell, allows to capture optical properties of each individual cell, contrary of larger systems, where many cells are analysed at same time, and outputs the mean optical properties of all cells, becoming impossible to distinguish and identify cells.

The lab-on-a-chip device can analyse phytoplankton cells *in-vivo* and *in-situ* conditions, thus the extraction of pigments from cells with solvents is not necessary, which is an advantage over other techniques.

In an embodiment, the device includes a lab-on-a-chip, with an inlet, an outlet and a constriction channel between both, that sorts phytoplankton cells to pass individually in the constriction channel, in front of the light path generated by the light source.

In an embodiment the lab-on-a-chip device includes 4 to 12 measurement lines. In a further embodiment, cells are illuminated in each line at specified wavelength, from 300 nm to 750 nm. Light transmission, scattering and fluorescence are measured, in the same or at different wavelength of the light source used to illuminate the cells.

In an embodiment, each measurement line comprises a light source that operates in the range between 300 nm to 750 nm; 3 photodiodes (transmission, scattering and fluorescence light); and an optical filter on top of each of the photodiodes.

In an embodiment, each measurement line comprises optical lens, mirrors, and waveguides to focus light of each light source into the cells passing in the constriction channel. Each light source illuminates the cell in a different wavelength, in the range of 300 nm to 750 nm. In a further embodiment, the optical lens and mirrors are fabricated in polydimethylsiloxane (PDMS) and reflective metal, such as gold, titanium, aluminium or silver, among others. They are used to focus the light in a single spot, located in the constriction channels where the cells are passing, with diameter between 5 $\mu$m and 50 $\mu$m, preferably 7-20 $\mu$m.

In an embodiment, each measurement line includes waveguides, fabricated in polydimethylsiloxane (PDMS) or epoxy-based negative photoresist (SU-8), and reflective metal, such as gold, titanium, aluminium or silver, among others, that are used to conduct light from the light source to the cells in the channel. In a further embodiment, the waveguides are used to conduct the light emitted from the phytoplankton cell to the 3 photodiodes of each measurement line.

In an embodiment, each measurement line includes a photodiode in front of the light source, used to measure transmitted light.

In a further embodiment, each measurement line comprises a photodiode at 90° anticlockwise from the direction of the light of the light source (in the top of lab-on-a-chip), coupled with optical filters, and used to measure fluorescence light from the cell being analysed.

In an embodiment, each measurement line includes a photodiode at 90° clockwise from the direction of the light of the light source (in the bottom of lab-on-a-chip), that can be used to measure fluorescence light, or alternatively used to measure lateral dispersion (or scattering) light from the cells. In embodiments where this photodiode is used to measure fluorescence light, an optical filter exists in front of the photodiode.

In an embodiment, the lab-on-a-chip device can have from 4 to 12 measurement lines as described in the present disclosure.

An embodiment of the device includes an electronic circuit, with amplifiers, a microcontroller and batteries, to control the activation of the light source, to amplify the current of each photodiode, to read the photodiodes current values, to identify the phytoplankton cell and count the number of cells of each specie based on its optical characteristics.

In an embodiment, the electronic circuit has limit of detection and sensibility that allows *in-vivo* identification and quantification of phytoplankton cells, thus the extraction of pigments from cells with solvents is not necessary.

In an embodiment, the lab-on-a-chip device includes biofouling protection that allows *in-situ* long term identification and quantification of phytoplankton cells.

In an embodiment, the electronics circuits can be integrated in the lab-on-a-chip device. In another embodiment, the electronics can be outside of the lab-on-a-chip.

In an embodiment, the device includes lock-in amplifiers to actuate the LEDs or lasers at a fixed frequency and read synchronously the photodiodes, thus increasing signal-to-noise ratio and decrease limit of detection. In a further embodiment, the device comprises at least one transimpedance amplifier to convert the current of photodiodes to voltage.

The present disclosure relates to a lab-on-a-chip device for *in-situ* and *in-vivo* identification and quantification of phytoplankton cell comprising an inlet and an outlet; a constriction microchannel that allows the passage of one single cell through the microchannel; and at least four measurement lines configured to identify and quantify a pre-defined phytoplankton cell, wherein each line comprises: one light source, preferably LEDs or laser; at least three photodiodes; wherein at least 2 photodiodes are different and wherein the three photodiodes are selected from a fluorescence photodiode, scattering photodiode, and transmission photodiode; and at least one optical filter, wherein the optical filter is aligned with the fluorescent photodiode; wherein the scattering photodiode, and fluorescence photodiode are not aligned with the source light.

In an embodiment, the three photodiodes are a fluorescence photodiode, scattering photodiode, and transmission photodiode; preferably wherein the device further comprising an electronic circuit for controlling the light sources and said electronic circuit comprises at least one amplifier, a microcontroller and batteries.

In an embodiment, the optical filter is selected from an optical filter for fluorescence analysis, an optical filter for scattering analysis, or an optical filter for optical transmission analysis.

In an embodiment, the lab-on-a-chip further comprises an electronic circuit. In a further embodiment, the electronic circuit comprises at least one amplifier, a microcontroller and batteries. In a yet further embodiment, the electronic circuit controls the light sources.

In an embodiment, the lab-on-a-chip device further comprises at least one transimpedance amplifier configured to convert the current of photodiodes into voltage.

In an embodiment, the lab-on-a-chip device further comprises at least one lock-in amplifier configured to excite the light source at a fixed frequency and read synchronously the photodiodes.

In an embodiment, the constriction channel has a width equal or higher than $5\mu$lm, preferably $5\mu$m to $50\ \mu$m, more preferably 5 to 20 $\mu$m.

In an embodiment, the photodiode and the optical filter for optical transmission analysis are placed frontally to the light source beam.

The device according to any of the previous claim, wherein the optical filter is placed in between the photodiode and the constriction channel.

In an embodiment, one photodiode and one optical filter for fluorescence analysis are placed at 90° anticlockwise from the direction of the light source beam.

In a further embodiment, one scattering photodiode or one fluorescent photodiode and one optical filter for fluorescence analysis are placed at 90° clockwise from the direction of the light source beam.

In an embodiment, the optical filter is placed in between the photodiode and the constriction channel.

In an embodiment, the lab-on-a-chip device further comprises optical lens, mirrors, and waveguides to focus the light emitted from the light source into a single spot within the constriction channel.

In an embodiment, the single spot has a diameter ranging from 5 $\mu$m to 50 $\mu$m, preferably 7-20 $\mu$m.

In an embodiment, the optical lens, mirrors and waveguides are fabricated in polydimethylsiloxane and reflective material, preferably wherein the reflective material is gold, titanium, aluminium, silver or combinations thereof. Physical vapor deposition (PVD) and Chemical vapor deposition (CVD) techniques can be used for reflective material deposition in the polydimethylsiloxane. In particular, the reflective material induces a series of light reflexions, which are crucial for the correct light guidance between the light source, the phytoplankton cells and the photodiodes.

In an embodiment, the light source emits light with a wavelength ranging from 300-700 nm.

In an embodiment, the lab-on-a-chip device described in the present disclosure further comprises an anti-biofouling agent selected from electrochlorination biocide generation system, UV irradiations, cooper compounds or mixtures thereof.

In an embodiment, the device comprises 4 to 12 measurement lines.

In an embodiment, the constriction channel is positioned between the inlet and the outlet.

The present disclosure also relates to a method to identify and quantify phytoplankton cells using the lab-on-a-chip device of the present disclosure, the method comprising the steps of:

> flowing the water sample through a constriction channel;
> activating light sources in a sequent manner and illuminating the phytoplankton cells;
> detecting and measuring optical properties of the phytoplankton using photodiodes of the lab-on-a-chip;
> identifying the phytoplankton cell by analysing the optical properties obtained;
> releasing the water sample to the external environment through the lab-on-a-chip outlet.

In an embodiment, the light sources have wavelengths of 320 nm, 420 nm, 450 nm and 530 nm.

In an embodiment, the optical properties are fluorescence, scattering and transmission properties of the phytoplankton cells.

In an embodiment, a programable micropump connected to the inlet of the lab-on-a-chip is used to collect/introduce the water sample, preferably seawater, and to control the flow inside the microchannel. The sample flows by the constriction region of the microchannel that allows the phytoplankton cells to move in a single centre line and pass one by one through the detection areas. In these areas, light sources with different wavelengths (320, 420, 450 and 530 nm) are sequentially activated to illuminate the cells. Its fluorescence, scattering and transmission properties are measured by the photodiodes. Each photodiode outputs a current value, which is amplified, converted to a digital value, by means of data acquisition electronics, and stored in the microcontroller for further calculations. After, the proposed methodology/algorithm implemented in the microcontroller runs and an automatic identification of the phytoplankton cell is achieved. Finally, the sample is released to the sea through the outlet of the lab-on-a-chip.

Another aspect of the invention relates to a kit or measuring equipment comprising the lab-on-a-chip device described previously.

**BRIEF DESCRIPTION OF THE DRAWINGS**

The following figures provide preferred embodiments for illustrating the disclosure and should not be seen as limiting the scope of invention.

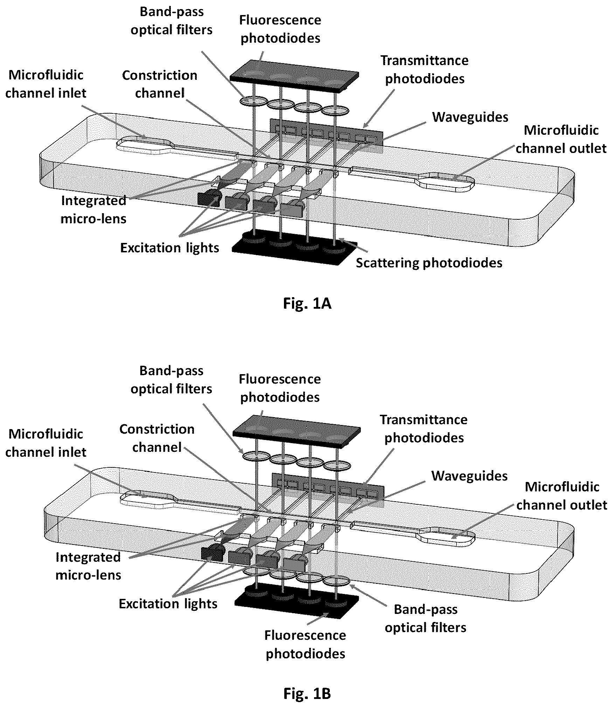

> **Figure 1:** Schematic representation of an embodiment of the device for phytoplankton identification and quantification. In this embodiment, 4 measurement lines are presented, but an embodiment can have from 4 to 12 measurement lines. In this embodiment, 4 light sources emitting light at different wavelengths are presented. The light from each LED or laser, is captured in 3 photodiodes. In front, for optical transmission of light, and at 90° of the light sources for fluorescence (Figure 1B) or lateral scattering of light **(Figure 1A)**. In this embodiment, a total of 12 photodiodes are used, grouped in the four measurement lines. **Figure 1A** represents an embodiment that uses the bottom photodiodes for lateral scattering, and no filters are required in these photodiodes. In a different embodiment **(Figure 1B),** the bottom photodiodes can also be used for fluorescence measurement, and filters are added on top of the photodiodes.

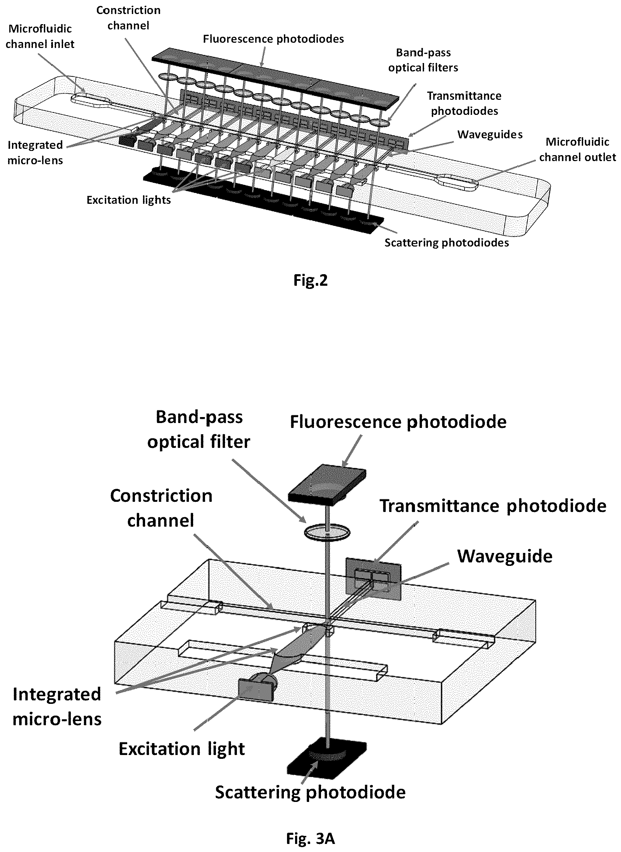

**Figure 2:** Schematic representation of an embodiment of the device for phytoplankton identification and quantification. In this embodiment, 12 measurement lines are used, but the device can include from 4 to 12 measurement lines. Each of the 12 lines of this embodiment includes a light source. The light from the light source, is captured in 3 photodiodes. Photodiodes in front, for optical transmission of light, and at 90° of the light source for fluorescence or lateral scattering of light. The photodiodes at 90° used to measure fluorescence use an optical filter to block light from the light source and allow the transmission of fluorescence light from the cell to the photodiode.

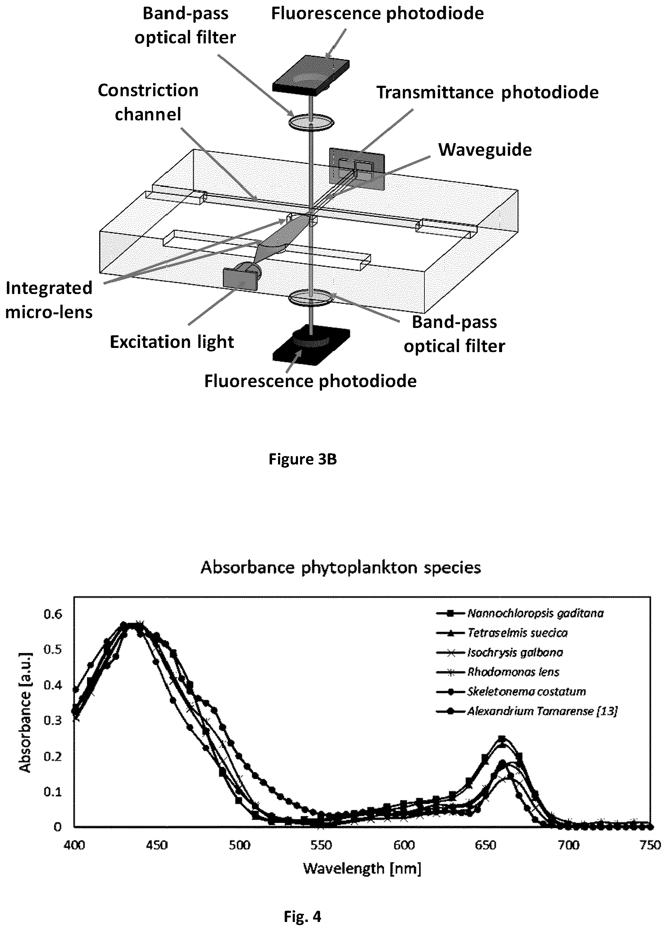

**Figure 3:** Detail representation of an embodiment of one measurement line. The measurement line comprises a light source for illumination; optical lens to focus the light; waveguides to conduct light to the cell, and from the cell to photodiodes; 3 photodiodes; and optical filters. In front, for optical transmission of light; at 90° of the light sources for fluorescence; and the third photodiode can be used to measure lateral scattering of light **(Figure 3A)** or fluorescence, whereas an optical filter is placed in between the light path and the photodiode placed at 90° clockwise to the light path **(Figure 3B).**

**Figure 4:** Illustration of an absorbance spectrum results (current in photodiode in front of the light source) demonstrating the possibility of identification of 6 different phytoplankton cells when wavelengths are carefully selected.

**Figure 5:** Scattering spectrum of *Nannochloropsis gaditana, Isochrysis galbana* and *Tetraselmis suecica,* with excitation light scanned from 600 nm to 700 nm, showing dispersion differences at 680 nm.

**Figure 6:** Illustration of an example of fluorescence (current in photodiode at 90° from the light source), when the phytoplankton cell *Nannochloropsis gaditana* is illuminated (excited) in the range between 320 nm and 700 nm.

**Figure 7:** Illustration of an example of fluorescence (current in photodiode at 90° from the light source), when the phytoplankton cell *Isochrysis galbana* is illuminated (excited) in the range between 320 nm and 700 nm.

**Figure 8:** Illustration of an example of fluorescence (current in photodiode at 90° from the light source), when the phytoplankton cell *Tetraselmis suecica* is illuminated (excited) in the range between 320nm and 700nm.

**Figure 9:** Illustration of the fluorescence obtained at 640 nm and at 685 nm (current in photodiode at 90° from the light source), when excitation light is scanned from 320 nm to 700 nm, in an embodiment to distinguish between *Nannochloropsis gaditana, Isochrysis galbana* and *Tetraselmis suecica* phytoplankton species **(Figure 9A)** and *Alexandrium Tamarense* (toxic specie) fluorescence spectrum with several excitations' wavelengths **(Figure 9B).**

**Figure 10:** Illustration of the fluorescence obtained in the range of 600 nm to 750 nm (current in photodiode at 90° from the light source), with an excitation light fixed at 420 nm **(Figure 10A),** 450 nm **(Figure 10B)** and 530 nm **(Figure 10C),** to select the wavelengths to be used in an embodiment to distinguish between *Nannochloropsis gaditana, Isochrysis galbana* and *Tetraselmis suecica* phytoplankton species.

**Figure 11:** Illustration of the obtained lateral scattering intensity from a 90° photodiode (x-axis) and forward light intensity from the transmission photodiode (y axis), in a measurement line with excitation at 488 nm, in an embodiment to distinguish between a) *Nannochloropsis gaditana;* b) *Isochrysis galbana;* c) *Tetraselmis suecica;* d) *Rhodomonas lens;* e) *Skeletonema costatum;* f) *Emiliania huxleyi.* phytoplankton species.

**Figure 12:** Schematic representation of an embodiment of a lock-in amplifier.

**Figure 13:** Embodiment of a calibration curve of the chlorophyll sensor using standard chlorophyll solutions.

**Figure 14:** Embodiment of a measurement of in vivo chlorophyll-a fluorescence from *Tetraselmis suecica* and *Skeletonema costatum* species diluted in seawater with lab-on-a-chip chlorophyll sensor (chlorophyll concentration in the range 0.02-250 µg/L).

**Figure 15:** Embodiment of a measurement of diluted seawater samples, with chlorophyll concentration in the range 0.05-2 µg/L, using the lab-on-a-chip chlorophyll sensor.

## DETAILED DESCRIPTION

The present disclosure relates to a lab-on-a-chip device used for identification and quantification of phytoplankton cells, in-situ and in-vivo, comprising an inlet and an outlet, a constriction channel, where the cells are forced to pass, an at least four measurement lines, each one comprising a light source, preferably LEDs or laser, three photodiodes and at least one optical filter for fluorescence analysis, dispersion analysis, or optical transmission analysis.

The device allows the simultaneous quantification and classification of phytoplankton species, that can be performed in a continuous and autonomous process. Also, the lab-on-a-chip described in the present disclosure is portable and can be used underwater. The fabrication process is compatible with low-cost policies, since all the components can be fabricated in poly(dimethylsiloxane) (PDMS). This material has optical transparency, needed for optical detection; allows the fabrication of nanometre features without the need of cleanroom facilities; and permits a low-cost prototyping [12].

In an embodiment, the lab-on-a-chip device has an inlet, a constriction channel and an outlet. Water containing phytoplankton cells enters in the lab-on-a-chip device by the inlet; is forced to flow in the constriction channel, and returns to the external environment through the outlet, as illustrated in **Figure 1A, Figure 1B and Figure 2.**

In a preferred embodiment, the constriction channel has a width related to the phytoplankton cell dimensions, preferred in the range of 5µm to 50 µm, preferably 5 to 20 µm but not limited to these dimensions, to force the cells to pass individually in the channel.

In an aspect of the present disclosure, the cells passing in the channel are illuminated by a LED or laser light, with light spot dimensions in the same range of the cells, preferably in the size range from 5 µm to 50 µm, preferably 7-20 µm. The device can contain several LEDs or lasers, preferably from 4 to 12, but not limited to 12, as illustrated in **Figures 1A and 1B** and **Figure 2.**

In an embodiment, each LED or laser in the lab-on-a-chip implements a measurement line, as illustrated in **Figure 3A and Figure 3B,** in conjunction with optical lens and optical waveguides fabricated in the lab-on-a-chip, in front of each LED or laser, that can focus the light in the described spot, and with 3 photodiodes that convert light into an electrical current.

In a preferred embodiment, the lab-on-a-chip device implements preferably 4 to 12 measurement lines, but can also have more measurement lines, and each measurement line can include: a light source, preferably a LED or laser, to illuminate the passing phytoplankton cells; an optical lens to focus the light in the cell; waveguides to transmit the light from the LED or laser to the cell; waveguides to transmit the light from the cell to the photodiodes; and 3 photodiodes to collect the light from the cell, that can have optical filters on top, when photodiodes are used to measure fluorescence.

An aspect of the present disclosure relates to the use of 3 photodiodes in each measurement line, respectively to measure transmitted light, with the photodiode in front of the light source; to measure fluorescence from the phytoplankton cell with a second photodiode placed at 90° anti-clockwise to the source of light, that includes an optical filter to block the light in the wavelength of the light source while allowing the passage of light with other wavelength that is emitted from the cell; and to measure lateral scattered light of the cell, with a third photodiode placed at 90° to the light source, symmetrically to the second photodiode relative to the light path.

Another embodiment of the present disclosure relates to the use of the third photodiode, placed at 90° from the light source symmetrically to the second photodiode relative to the light path, to measure fluorescence, instead of measuring lateral scattered light, by adding also an optical filter to block the light in the wavelength of the LED or laser, while allowing the passage of light with other wavelength, that is emitted from the cell.

An embodiment includes 4 to 10 measurement lines, and in each measurement line, the third photodiode (placed at 90° from the light source) can be used or for scattering light measurement or for fluorescence measurement.

The present disclosure relates to a lab-on-a-chip device that measures the optical properties (transmission or absorption; lateral dispersion; and fluorescence) of the cells in several wavelengths, comprising several measurement lines.

The absorption of light from six different phytoplankton species *(Nannochloropsis gaditana, Tetraselmis suecica, Isochrysis galbana, Rhodomonas lens, Skeletonema costatum* and *Alexandrium tamarense* [13]) is illustrated in **Figure 4,** demonstrating that optical transmission (or the inverse, absorption) can be used to differentiate phytoplankton cells. Each specie presents a distinct absorption spectrum, the combination of the multiple photodiodes allows the precise specie identification.

In an embodiment after entering in the device via the inlet, the phytoplankton cells are forced to pass through the constriction channel until reaching the outlet. During the passage, cells are illuminated by light sources with a specific wavelength. The presence of optical lens, mirrors and waveguides assures that the light emitted from the light source is focused into a single spot within the constriction channel, illuminating only one phytoplankton cell at a time.

In an embodiment, after being illuminated by a specific wavelength, the cell can enter in an excited state, absorb or transmit light. The resulting light, i.e. transmitted or emitted light, is conducted from the cell to a photodiode, that transforms the light energy in an electric current. The resulting electric current is transmitted to an electronic circuit, that can be embedded in the lab-on-a-chip device or placed externally to the device. The electronic circuit comprises a microcontroller unit, capable of identifying and counting the number of phytoplankton cells of each species, that pass through the constriction channel.

In an embodiment, the lab-on-a-chip device includes also a battery for power, micropumps and a microcontroller for data storing and for controlling the lab-on-a-chip operation, that can be embedded or placed externally to the device.

The following example illustrates an embodiment of the lab-on-a-chip device described on the present disclosure.

### Example 1:

In an embodiment, the optical properties that can be used to distinguish species of phytoplankton according to single characteristics were considered. Particularly, absorption and dispersion spectrum, and fluorescence in the range of 600 nm to 750 nm, with excitation in the range of 320 nm to 700 nm.

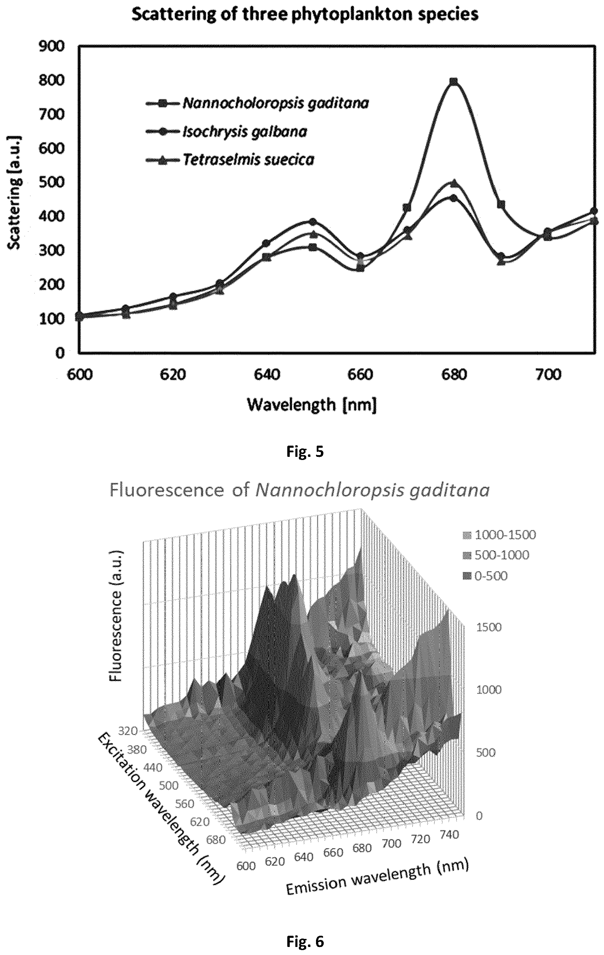

Surprisingly, it was found that the relationship between the absorption coefficient at 660 nm and 430 nm, obtained from a spectroscopic analysis **(Figure 4),** can be used to distinguish phytoplankton species (Table 1). Also, light scattering of a phytoplankton samples (600 nm to 700 nm) **(Figure 5)** can also be used for identifications purposes. Surprisingly, it was noticed that species of phytoplankton can be grouped by their colour, analysing their absorption and scattering spectra.

Table 1 - Relation between absorption at 660 nm and 430 nm of the phytoplankton species.

| Specie | $A_{660}/A_{430}$ |
|---|---|
| *Nannochloropsis gaditana* | *0.440* |
| *Isochrysis galbana* | *0.419* |
| *Tetraselmis suecica* | *0.244* |
| *Rhodomonas lens* | *0.311* |
| *Skeletonema costatum* | *0.298* |
| *Alexandrium tamarense* | *0.336* |

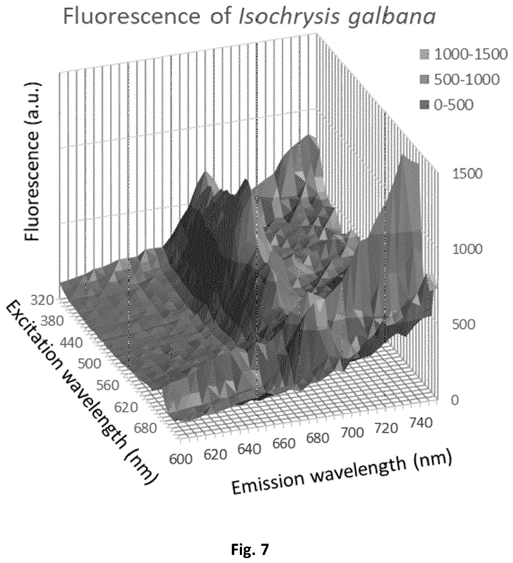

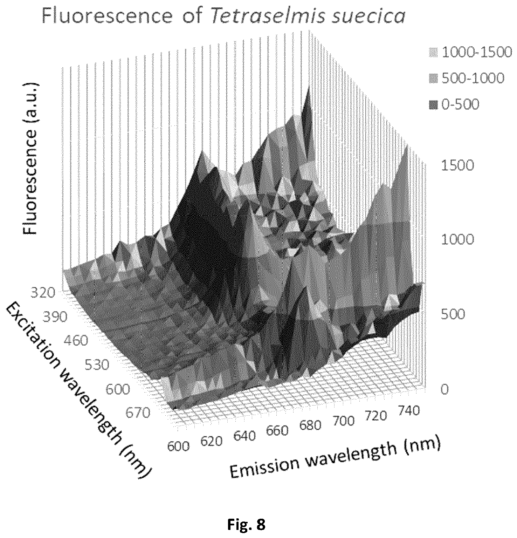

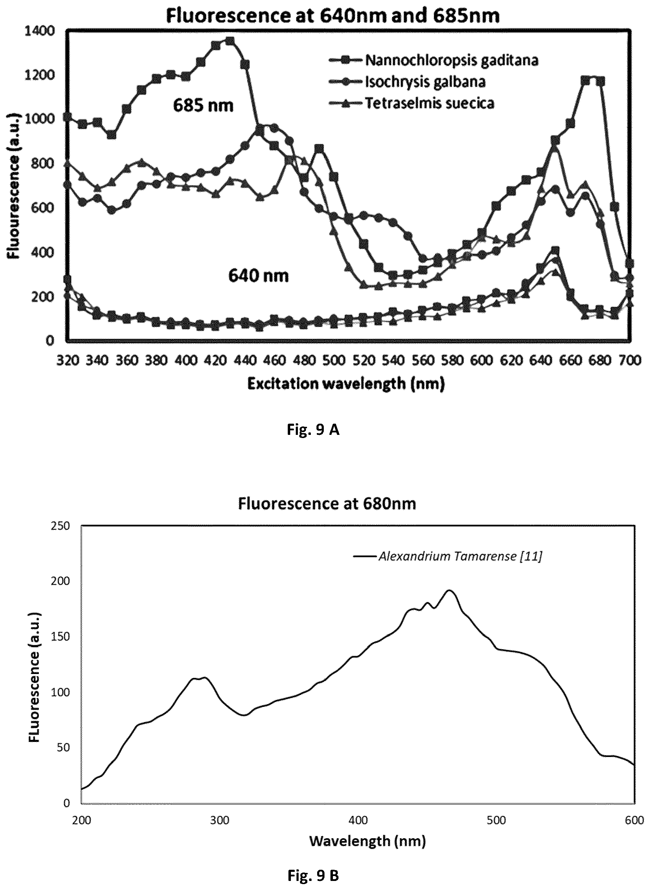

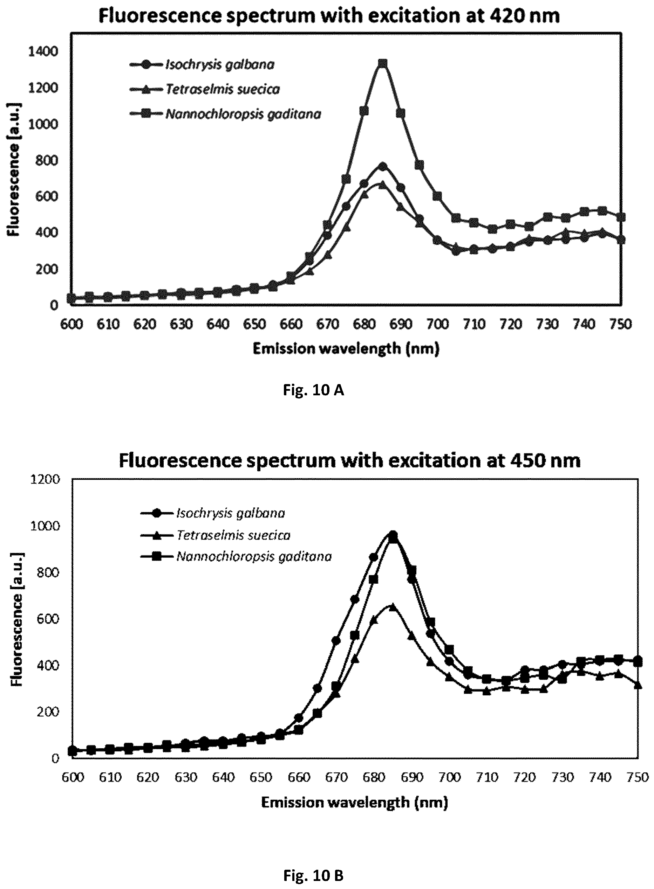

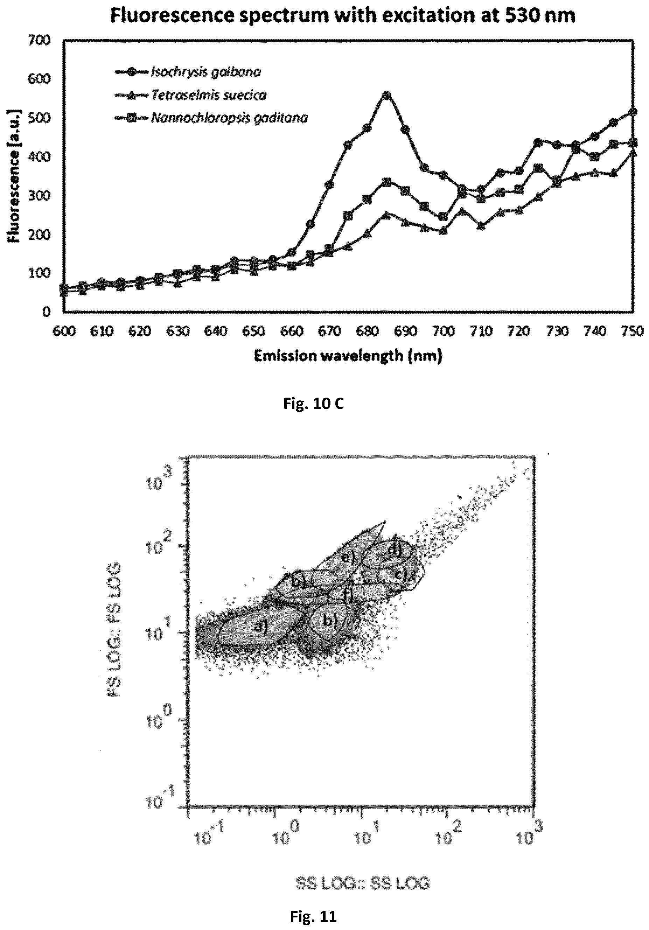

In a further embodiment, fluorometric measurements can be used to complement the identification via analysis of the absorption and dispersion spectra. The excitation wavelengths of 320 nm, 420 nm, 450 nm and 530 nm **(Figure 10A, Figure 10B and Figure 10C)** and the emission wavelengths of 640 nm and 685 nm were selected to distinguish the different phytoplankton species **(Figure 9A and Figure 9B).** Hence, a lab-on-a-chip device with four measurement lines was used to distinguish different species of phytoplankton. The light sources used in this embodiment emitted a light with 320, 420, 450 and 530 nm, respectively. Table 2 describes different embodiments of pairs of excitation/emission wavelengths used to distinguish non-toxic species (*Nannochloropsis gaditana, Isochrysis galbana* and *Tetraselmis suecica*) and toxic species (*Alexandrium tamarense*) and respective measured fluorescence value **(Figure 6, Figure 7, Figure 8).**

Table 2 - Pairs of excitation/emission wavelengths used to distinguish *Nannochloropsis gaditana, Isochrysis galbana Tetraselmis suecica* and *Alexandrium tamarense* and respective measured fluorescence value.

| Fluorescence of *Nannochloropsis gaditana* (a.u.) | Emission (nm) | |
|---|---|---|
| Excitation (nm) | X: 685 | Y: 640 |
| A: 320 | 1010 | |

(continued)

| Fluorescence of *Nannochloropsis gaditana* (a.u.) | Emission (nm) | |
|---|---|---|
| Excitation (nm) | X: 685 | Y: 640 |
| B: 420 | 1332 | 74 |
| C: 450 | 944 | 71 |
| D: 530 | 335 | 109 |
| Fluorescence of *Isochrysis galbana* (a.u.) | Emission (nm) | |
| Excitation (nm) | X: 685 | Y: 640 |
| A: 320 | 707 | |
| B: 420 | 768 | 74 |
| C: 450 | 962 | 75 |
| D: 530 | 533 | 110 |
| Fluorescence of *Tetraselmis suecica* (a.u.) | Emission (nm) | |
| Excitation (nm) | X: 685 [11] | Y: 640 [14] |
| A: 320 | 805 | |
| B: 420 | 665 | 66 |
| C: 450 | 653 | 62 |
| D: 530 | 251 | 90 |
| Fluorescence of *Alexandrium tamarense* (a.u.) | Emission (nm) | |
| Excitation (nm) | X: 685 | Y: 640 |
| A: 320 | 79,84 | |
| B: 420 | 150,4 | 8 |
| C: 450 | 180,8 | 10 |
| D: 530 | 128,8 | 9 |

In a further embodiment, for each specie, nine ratios between the fluorescence values of Table 3 were defined: six ratios between the 4 fluorescence values obtained at 685 nm, with the 4 different excitation wavelengths (320 nm, 420 nm, 450 nm and 530 nm), named $F_{BX/CX}$, $F_{BX/DX}$ $F_{BX/AX}$, $F_{CX/DX}$, $F_{CX/AX}$, $F_{DX/AX}$ ; and three ratios between the emission obtained at 685 nm and 640 nm, for each excitation wavelengths (320 nm, 420 nm, 450 nm and 530 nm), named $F_{BX/BY}$, $F_{CX/CY}$ $F_{DX/DY}$. These ratios are presented in Table 3, for each specie:

Table 3. Fluorescence ratios used to distinguish *Nannochloropsis gaditana*, *Isochrysis galbana*, *Tetraselmis suecica* and *Alexandrium tamarense*.

| Ratio | Specie | | | |
|---|---|---|---|---|
| | Nannochloropsis gaditana (GAD) | Isochrysis galbana (GAL) | Tetraselmis suecica (SUE) | Alexandrium tamarense (TAM) |
| $F_{BX/CX}$ | 1.41 (M) | 0.80 (m) | 1.02 | 0.83 |
| $F_{BX/DX}$ | 3.98 (M) | 1.44 | 2.65 | 1.17 (m) |
| $F_{BX/AX}$ | 1.32 | 1.09 | 0.83 (m) | 1.88 (M) |
| $F_{CX/DX}$ | 2.82 (M) | 1.80 | 2.60 | 1.40 (m) |
| $F_{CX/AX}$ | 0.93 | 1.36 | 0.81 (m) | 2.26 (M) |
| $F_{DX/AX}$ | 0.33 | 0.75 | 0.31 (m) | 1.61 (M) |

(continued)

| Ratio | Specie | | | |
|---|---|---|---|---|
| | Nannochloropsis gaditana (GAD) | Isochrysis galbana (GAL) | Tetraselmis suecica (SUE) | Alexandrium tamarense (TAM) |
| $F_{BX/BY}$ | 18 | 10.38 | 10.08 (m) | 18.80 (M) |
| $F_{CX/CY}$ | 13.30 | 12.83 | 10.53 (m) | 18.08 (M) |
| $F_{DX/DY}$ | 3.07 | 4.85 | 2.79 (m) | 14.31 (M) |

In an embodiment, for each ratio listed in Table 3, the maximum (M) and minimum (m) values (maximum and minimum at each row) were selected. Maximum values and minimum values of fluorescence ratios were then used as identifying characteristics of each specie. These values were then used to calculate an identifier for each specie (GAD, GAL, SUE and TAM), using Equation 1. Maximum ratios from Table 3 are in numerator and minimum ratios in denominator of equations to calculate GAD, GAL, SUE and TAM of Eq. 1. Other fluorescence ratios were also selected to improve differentiation in these 4 calculated identifiers. A coefficient was also considered, respectively 1/284.57, 1/0.24, 61.8 and 1/20423, only to normalize all identifiers to a maximum of 1.

$$GAD = \frac{F_{BX/CX} \times F_{BX/DX} \times F_{CX/DX} \times F_{BX/BY}}{284.57} \qquad \text{Eq. 1.1}$$

$$GAL = \frac{F_{CX/CY} \times F_{BX/AX}}{0.24 \times F_{BX/CX} \times F_{BX/DX} \times F_{BX/BY} \times F_{DX/DY}} \qquad \text{Eq. 1.2}$$

$$SUE = \frac{61.8}{F_{BX/AX} \times F_{CX/AX} \times F_{DX/AX} \times F_{BX/BY} \times F_{CX/CY} \times F_{DX/DY}} \qquad \text{Eq. 1.3}$$

$$TAM = \frac{F_{BX/AX} \times F_{CX/AX} \times F_{DX/AX} \times F_{BX/BY} \times F_{CX/CY} \times F_{DX/DY}}{20423 \times F_{BX/DX} \times F_{CX/DX}} \qquad \text{Eq. 1.4}$$

In an embodiment, the equations were validated by calculating the four identifiers using the fluorescence values of the four species (Table 3). The results are listed in Table 4.

Table 4: Four identifiers (GAD, GAL, SUE and TAM) calculated from fluorescence ratios of the three species.

| Species | GAD | GAL | SUE | TAM |
|---|---|---|---|---|
| Nannochloropsis gaditana | **1.00** | 0.08 | 0.25 | 0.09 |
| Isochrysis galbana | 0.24 | **1.00** | 0.48 | 0.54 |
| Tetraselmis suecica | 0.21 | 0.09 | **1.00** | 0.00 |
| Alexandrium tamarense | 0.00 | 0.01 | 0.00 | **1.00** |

In an embodiment, an identifying algorithm calculates the value of the four identifiers (GAL, GAD, SUE and TAM) and the higher value obtained reveals the presented specie.

In a further embodiment, if other species are presented for identification, the same methodology can be used. However, more emission ratios (than the ones presented in Table 3) should be used, in order to improve the selectivity of the identification process.

In an embodiment, complementing the lab-on-a-chip with forward scattering and side scattering, improves the identification process. A lab-on-a-chip that measures fluorescence in four wavelengths (such as FL1-525nm, FL2-575nm, FL3-620nm and FL4-675nm) can be used to distinguish between phytoplankton species, except those from the same group, that are identified by using the other optical techniques (absorption, scattering, fluorescence).

Figure 1A and 1B depicts a schematic representation of an embodiment of the lab-on-a-chip with integrated fluorescence and scattering detection (lateral scattering). This system can be constituted of a microfluidic die, which includes microchannels and a detection chamber; a set of excitation light sources, with specific wavelengths for the different species of phytoplankton; CMOS silicon photodiodes; signal amplifiers; data acquisition for analog to digital conversion; battery; micropumps; and a microcontroller. All of these parts are integrated in a portable and compact platform.

In an embodiment **(Figure 1A),** the lab-on-a-chip was designed with four excitation wavelengths (320 nm, 420 nm, 450 nm, and 530 nm) that can be obtained with LEDs or lasers (light sources); four photodiodes, for fluorescence light, wherein light passes through an optical filter before reaching the photodiode; four photodiodes for scattered light, and four photodiodes for transmitted light. Besides the light sources and photodiodes, the lab-on-a-chip of the present disclosure comprises a constriction channel, in order to analyse single cells (or a few cells), avoiding the measurement of mean values of the optical properties.

In a different embodiment **(Figure 1B),** a lab-on-a-chip for phytoplankton classification was designed with four excitation wavelengths (320 nm, 420 nm, 450 nm, and 530 nm) that can be obtained with LEDs or lasers (light sources); suitable optical filters for emission wavelengths (640 nm and 685 nm); and twelve photodiodes (four fluorescence photodiodes with an optical filter at 640 nm, four fluorescence photodiodes with an optical filter at 685 nm, and four photodiodes for transmitted light). Besides the light sources and photodiodes, the lab-on-a-chip of the present disclosure comprises a constriction channel, in order to analyse single cells (or a few cells), avoiding the measurement of mean values of the optical properties.

In an embodiment, the microfluidic die was designed to lead the phytoplankton cells into the detection channel. This microfluidic die was fabricated in polydimethylsiloxane (PDMS) due to its advantages such as flexibility, biocompatibility and optical transparency in the visible/ultra-violet (down to 230 nm) spectral range, which allows the use of optical detection methods. The fabrication of the PDMS microfluidic die was performed using a low-cost method, without the need of cleanroom facilities. The PDMS was cast using a SU-8 mould (soft lithography) obtained by a high-resolution photolithography process. In order to close the microchannels (top and bottom) a $O_2$ plasma treatment was performed during 30 seconds with 30 W power, to bond the PDMS microchannels with a PDMS layer.

In a further embodiment, four micro LEDs were chosen as excitation light source, with wavelengths of 320, 420, 450 and 530 nm. Lens were fabricated in the PDMS die, in order to focus the light in a small spot, in the constriction channel. Eight photodiodes (with band-pass filters at 640 nm and 680 nm) were placed at a 90° angle (clockwise and anticlockwise) from the LEDs, to measure fluorescence and minimize influence of excitation light. Four photodiodes were also added in front of the illumination sources, one photodiode per illumination source, to measure the transmitted light. Waveguides were used to conduct the transmitted light from the cell to the photodiodes. Dichroic mirrors can also be used in fluorescence analysis at several wavelengths using a single excitation light, allowing a selective passage of light within a small range of wavelengths (colours), while reflecting other wavelengths.

In an embodiment, **Figure 11** illustrates the lateral scattering intensity from a 90° photodiode (x-axis) and forward light intensity from the transmission photodiode (y axis), in a measurement line with excitation at 488 nm. In this embodiment the species are distinguished, being a) *Nannochloropsis gaditana;* b) *Isochrysis galbana;* c) *Tetraselmis suecica;* d) *Rhodomonas lens;* e) *Skeletonema costatum;* f) *Emiliania huxleyi.* phytoplankton species.

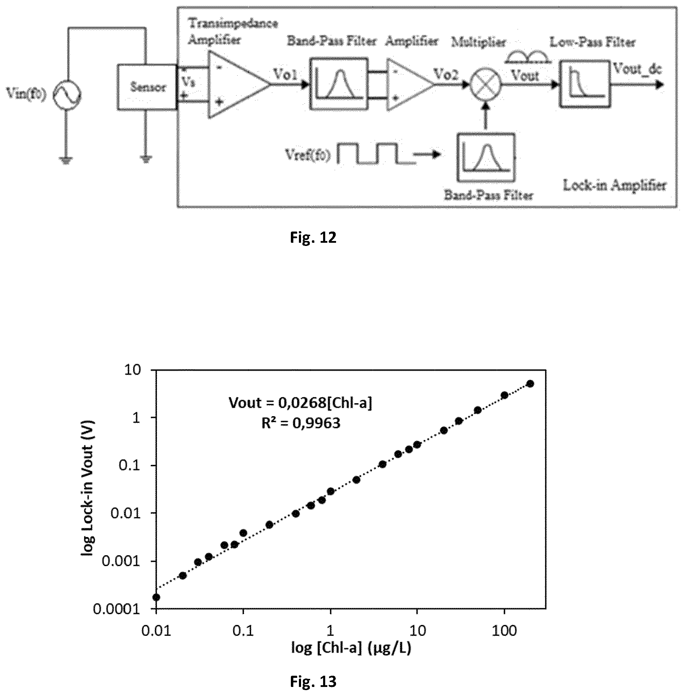

In a yet further embodiment, it was implemented a high-sensitivity, noise-free reading circuit capable of making viable fluorescence readings. Typically, the fluorescence signal has a low amplitude, consequently resulting in a low current in the photodiodes (in nA magnitude for low chlorophyll concentrations). A lock-in amplifier was implemented using synchronous detectors. It extracts the signal embedded in noise, allowing a higher signal-to-noise ratio, since the noise decreases as the frequency increases. Shifting the useful detection signal to higher frequencies, the signal-to-noise ratio is increased, allowing the detection of signals of smaller frequency, such as expected in phytoplankton fluorescence [15]. This was achieved by modulating the excitation light (LED or laser). There are several methods available in the state-of-art to modulate the LED or laser. In an embodiment, the modulation was performed by pulsing it in the order of kHz, shifting the signal of interest to frequencies where lower noise is expected. The original DC signal (from fluorescence) was recovered through a synchronous demodulator that shifted the signal from a few kHz to DC, rejecting all signals that are not synchronized with the excitation reference. A representative embodiment of a block diagram of a lock-in amplifier is shown **in Figure 12.**

In an embodiment, a proof-of-concept prototype of the miniaturized and low-cost fluorescent detection system was fabricated using 4 subsystems:

1 - the illumination system containing commercial LEDs with maximum emission peaks centred at 450 nm;
2 - the photodetection system containing a low-noise and high quantum efficiency photodiodes with a 600 nm optical long-pass bandpass filter for supressing the excitation signal and improve the sensitivity by drastically reducing the background noise;
3 - power source batteries; and
4 - the electronic system for readout and control. The optical-electronic system was assembled in a 90° configuration using a 3D printed support that ensures the correct alignment and position of the optical components.

Furthermore, it was designed with dimensions suitable for analyse in standard cuvettes. In this stage, the control and readout electronics was mounted in a PCB placed externally to the assembled device.

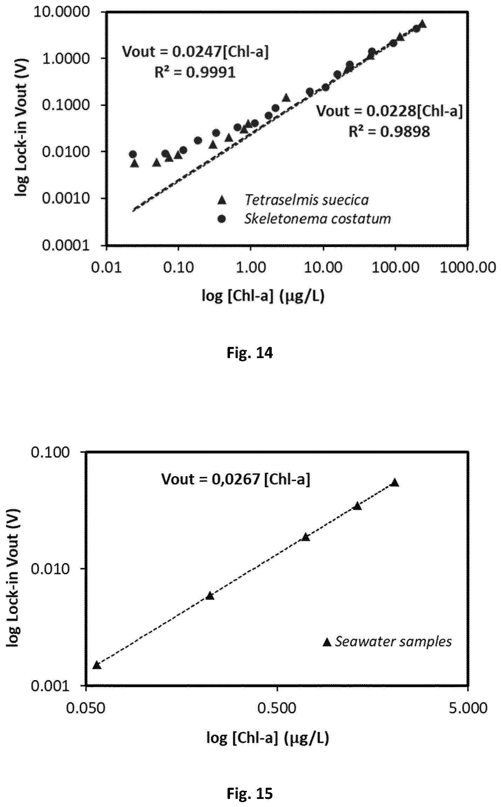

In a further embodiment, the device performance was measured, considering the limit of detection, measurement range, and linearity. Thus, the device's response was initially calibrated using chlorophyll-a standard solutions with known concentrations in the range of 0.01-300 $\mu$g/L. The sensor shows a linear response ($R^2$ = 0.9963) with a limit of detection of 0.01 $\mu$g/L using chlorophyll-a standards **(Figure 13).** After calibration, a test was performed with specific phytoplankton species (*Tetroselmis suecica* and *Skeletonema costatum).* Phytoplankton species were diluted in filtered seawater, with concentrations in the range of 0.02-250 $\mu$g/L. Each sample was measured with the chlorophyll sensor and the output voltage is showed in **Figure 14.** The sensor shows a linear response with a limit of detection of 0.05 $\mu$g/L using *in-vivo* samples of *Tetraselmis suecica* and *Skeletonema costatum* species.

In another embodiment, tests were performed with seawater, collected from Esposende, Portugal, 41.533156N, 8.784164W, containing several different phytoplankton species. Collected seawater was diluted with filtered seawater, in order to obtain 5 different water samples with chlorophyll concentration in the range 0.05 - 2 $\mu$g/L. This low concentration allowed to evaluate the limit of detection of the device. Measurement results are presented in **Figure 15.** The measurements realized with the developed device present an excellent performance, able to measure chlorophyll-a concentration bellow 0.1 $\mu$g/L, in ranges up to 300 $\mu$g/L.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

The following claims further set out particular embodiments of the disclosure.

The following references, should be considered herewith incorporated in their entirety:

[1] Lindsey, R. "What are Phytoplankton? : Feature Articles," 16-Jul-2010. [Online]. Available: https://earthobservatory.nasa.gov/Features/Phytoplankton/?src.

[2] Berdalet, E., Fleming, L. E., Gowen, R., Davidson, K., Hess, P., Backer, L. C. & Enevoldsen, H. (2016). Marine harmful algal blooms, human health and wellbeing: challenges and opportunities in the 21st century. Journal of the Marine Biological Association of the United Kingdom, 96(1), 61-91.

[3] Vale, P., Botelho, M. J., Rodrigues, S. M., Gomes, S. S. & Sampayo, M. A. (2008). Two decades of marine biotoxin monitoring in bivalves from Portugal (1986-2006): a review of exposure assessment. Harmful Algae, 7(1), 11-25.

[4] Zohdi, E., & Abbaspour, M. (2019). Harmful algal blooms (red tide): a review of causes, impacts and approaches to monitoring and prediction. International journal of environmental science and technology, 16(3), 1789-1806.

[5] Glibert, P. M., Pitcher, G. C., Bernard, S., & Li, M. (2018). Advancements and continuing challenges of emerging technologies and tools for detecting harmful algal blooms, their antecedent conditions and toxins, and applications in predictive models. In Global Ecology and Oceanography of Harmful Algal Blooms (pp. 339-357). Springer, Cham.

[6] Shin, Y. H., Barnett, J. Z., Gutierrez-Wing, M. T., Rusch, K. A., & Choi, J. W. (2018). A hand-held fluorescent sensor platform for selectively estimating green algae and cyanobacteria biomass. Sensors and Actuators B: Chemical, 262, 938-946.

[7] Speer, B. R. "Photosynthetic Pigments," University of California, Berkeley Museum of Paleontology, 1997. [Online]. Available: http://www.ucmp.berkeley.edu/glossary/gloss3/pigments.html.

[8] Gonçalves, S., Manual para a avaliação da qualidade biológica da água em Lagos e Albufeiras segundo a directiva quadro da agua, in Instituto da Água, I.P. 2009. p. 1-40.

[9] Johnson, Z.I. and Martiny, A.C. Techniques for quantifying phytoplankton biodiversity. 2015. 7: p. 299-324.

[10] Su, R., Chen, X., Wu, Z., Yao, P., & Shi, X. (2015). Assessment of phytoplankton class abundance using fluorescence excitation-emission matrix by parallel factor analysis and nonnegative least squares. Chinese journal of oceanology and limnology, 33(4), 878-889.

[11] Zhang, Q. Q., et al., Discrimination of phytoplankton classes using characteristic spectra of 3D fluorescence spectra. 2006. 63(2): p. 361-369.

[12] Pinto, V. C., Sousa, P. J., Cardoso, V. F., & Minas, G. (2014). Optimized SU-8 processing for low-cost microstructures fabrication without cleanroom facilities. Micromachines, 5(3), 738-755.

[13] He, H., Chen, F., Li, H., Xiang, W., Li, Y., & Jiang, Y. (2010). Effect of iron on growth, biochemical composition and paralytic shellfish poisoning toxins production of Alexandrium tamarense. Harmful Algae, 9(1), 98-104.

[14] Zhang, F., He, J., Su, R., & Wang, X. (2011). Assessing Phytoplankton Using a Two-Rank Database Based on Excitation-Emission Fluorescence Spectra. Applied spectroscopy, 65(1), 1-9.

[15] Maya-Hernández, P. M., Álvarez-Simón, L. C., Sanz-Pascual, M. T., & Calvo-Lopez, B. (2014). An integrated low-power lock-in amplifier and its application to gas detection. Sensors, 14(9), 15880-15899.
[16] Allison Schaap, et al.,"Optical classification of algae species with a glass lab-on-a-chip", Lab on a Chip; 27th January 2012 , DOI: 10.1039/c2lc21091f.

**Claims**

1. A lab-on-a-chip device for in-situ and in-vivo identification and quantification of phytoplankton cell comprising:

   an inlet and an outlet;
   a constriction microchannel that allows the passage of one single cell through the microchannel; and
   at least four measurement lines configured to identify and quantify a pre-defined phytoplankton cell, wherein each line comprises:

   one light source, preferably LEDs or laser;
   at least three photodiodes; wherein at least 2 photodiodes are different and
   wherein the three photodiodes are selected from a fluorescence photodiode,
   scattering photodiode, and transmission photodiode; and
   at least one optical filter, wherein the optical filter is aligned with the fluorescent photodiode;
   wherein the scattering photodiode, and fluorescence photodiode are not aligned with the source light.

2. The device according to any of the previous claim the three photodiodes are a fluorescence photodiode, scattering photodiode, and transmission photodiode; preferably wherein the device further comprising an electronic circuit for controlling the light sources and said electronic circuit comprises at least one amplifier, a microcontroller and batteries.

3. The device according to any of the previous claims wherein one photodiode and one optical filter for fluorescence analysis are placed at 90° anticlockwise from the direction of the light source beam.

4. The device according to any of the previous claims wherein one scattering photodiode or one fluorescent photodiode and one optical filter for fluorescence analysis are placed at 90° clockwise from the direction of the light source beam.

5. The device according to any of the previous claims further comprising at least one transimpedance amplifier configured to convert the current of photodiodes into voltage.

6. The device according to any of the previous claims further comprising at least one lock-in amplifier configured to excite the light source at a fixed frequency and read synchronously the photodiodes.

7. The device according to any of the previous claims wherein the constriction channel has a width equal or higher than $5\mu m$, preferably $5\mu m$ to $50\ \mu m$.

8. The device according to any of the previous claims wherein the photodiode and the optical filter for optical transmission analysis are placed frontally to the light beam.

9. The device according to any of the previous claim, wherein the optical filter is placed in between the photodiode and the constriction channel.

10. The device according to any of the previous claims further comprising optical lens, mirrors, and waveguides to focus the light emitted from the light source into a single spot within the constriction channel.

11. The device according to the previous claim wherein the single spot has a diameter ranging from $5\ \mu m$ to $50\ \mu m$, preferably $7-20\ \mu m$.

12. The device according to any of the previous claims wherein the light source emits light with a wavelength ranging from 300-700 nm, preferably 320 nm, 420 nm, 450 nm and 530 nm.

13. The device according to any of the previous claims further comprising an anti-biofouling agent selected from electrochlorination biocide generation system, UV irradiations, cooper compounds or mixtures thereof.

**14.** Method to identify and quantify phytoplankton cells using the lab-on-a-chip device described in any of the previous claims, the method comprising the steps of:

collecting a water sample containing phytoplankton cells using a micropump connected to the lab-on-a-chip inlet;
flowing the water sample through a constriction channel;
activating light sources in a sequent manner and illuminating the phytoplankton cells;
detecting and measuring optical properties of the phytoplankton using photodiodes of the lab-on-a-chip;
identifying the phytoplankton cell by analysing the optical properties obtained;
releasing the water sample to the external environment through the lab-on-a-chip outlet.

**15.** A kit or measuring equipment comprising the lab-on-a-chip device according to claims 1-13.

Band-pass
optical filters    Fluorescence
photodiodes

Transmittance
photodiodes

Microfluidic     Constriction
channel inlet    channel

Waveguides
Microfluidic
channel outlet

Integrated
micro-lens

Excitation lights

Scattering photodiodes

**Fig. 1A**

Band-pass
optical filters    Fluorescence
photodiodes

Transmittance
photodiodes

Microfluidic     Constriction
channel inlet    channel

Waveguides
Microfluidic
channel outlet

Integrated
micro-lens

Excitation lights

Band-pass
optical filters

Fluorescence
photodiodes

**Fig. 1B**

Microfluidic
channel inlet

Constriction
channel

Fluorescence photodiodes

Band-pass
optical filters

Transmittance
photodiodes

Integrated
micro-lens

Waveguides

Microfluidic
channel outlet

Excitation lights

Scattering photodiodes

**Fig.2**

Band-pass
optical filter

Fluorescence photodiode

Constriction
channel

Transmittance photodiode

Waveguide

Integrated
micro-lens

Excitation light

Scattering photodiode

**Fig. 3A**

**Figure 3B**

**Fig. 4**

## Scattering of three phytoplankton species

**Fig. 5**

## Fluorescence of *Nannochloropsis gaditana*

**Fig. 6**

Fluorescence of *Isochrysis galbana*

**Fig. 7**

Fluorescence of *Tetraselmis suecica*

Fig. 8

**Fluorescence at 640nm and 685nm**

Legend:
- Nannochloropsis gaditana
- Isochrysis galbana
- Tetraselmis suecica

685 nm

640 nm

Y-axis: Fluourescence (a.u.)
X-axis: Excitation wavelength (nm)

**Fig. 9 A**

**Fluorescence at 680nm**

Legend:
- Alexandrium Tamarense [11]

Y-axis: FLuorescence (a.u.)
X-axis: Wavelength (nm)

**Fig. 9 B**

## Fluorescence spectrum with excitation at 420 nm

Fig. 10 A

## Fluorescence spectrum with excitation at 450 nm

Fig. 10 B

## Fluorescence spectrum with excitation at 530 nm

Fig. 10 C

Fig. 11

**Fig. 12**

Vout = 0,0268[Chl-a]
R² = 0,9963

**Fig. 13**

**Fig. 14**

**Fig. 15**

**EP 3 943 918 A1**

Europäisches Patentamt

European Patent Office

Office européen des brevets

# EUROPEAN SEARCH REPORT

**Application Number**

EP 21 18 7313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NASTARAN HASHEMI ET AL: "Microflow Cytometer for optical analysis of phytoplankton", BIOSENSORS AND BIOELECTRONICS, ELSEVIER SCIENCE LTD, UK, AMSTERDAM , NL, vol. 26, no. 11, 31 March 2011 (2011-03-31), pages 4263-4269, XP028378349, ISSN: 0956-5663, DOI: 10.1016/J.BIOS.2011.03.042 [retrieved on 2011-04-30] * abstract; figure 1 * * page 6264 - page 6268; table 1 * | 1-15 | INV. G01N21/53 G01N21/63 G01N21/64 G01N21/49 B01L3/00 G01N33/18 |
| Y | US 2009/122311 A1 (KANDA MASAHIKO [JP]) 14 May 2009 (2009-05-14) * paragraphs [0003] - [0009]; figures 7,8; table 1 * | 1-15 | |
| Y | US 8 654 319 B2 (RAO GOVIND [US]; LAM HUNG [US] ET AL.) 18 February 2014 (2014-02-18) * column 4, lines 14-17 * | 13 | |
| A | US 6 592 822 B1 (CHANDLER VAN S [US]) 15 July 2003 (2003-07-15) * column 3, lines 1-10; figure 3 * | 1-15 | TECHNICAL FIELDS SEARCHED (IPC) G01N B01L |
| A,D | SCHAAP ALLISON ET AL: "Lab on a chip technologies for algae detection: a review", JOURNAL OF BIOPHOTONICS, vol. 5, no. 8-9, 13 June 2012 (2012-06-13) , pages 661-672, XP055861541, DE ISSN: 1864-063X, DOI: 10.1002/jbio.201200051 * the whole document * | 1-15 | |

The present search report has been drawn up for all claims

| Place of search | Date of completion of the search | Examiner |
|---|---|---|
| Munich | 17 November 2021 | Meacher, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 7313

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2021

| Patent document cited in search report | | Publication date | Patent family member(s) | | Publication date |
|---|---|---|---|---|---|
| US 2009122311 | A1 | 14-05-2009 | DE 112006002091 | T5 | 10-07-2008 |
| | | | JP 4756948 | B2 | 24-08-2011 |
| | | | JP 2007046947 | A | 22-02-2007 |
| | | | US 2009122311 | A1 | 14-05-2009 |
| | | | WO 2007018087 | A1 | 15-02-2007 |
| US 8654319 | B2 | 18-02-2014 | EP 2389447 | A1 | 30-11-2011 |
| | | | US 2011273705 | A1 | 10-11-2011 |
| | | | WO 2010085736 | A1 | 29-07-2010 |
| US 6592822 | B1 | 15-07-2003 | AU 3897999 | A | 29-11-1999 |
| | | | CA 2331897 | A1 | 18-11-1999 |
| | | | CA 2640578 | A1 | 18-11-1999 |
| | | | JP 4215397 | B2 | 28-01-2009 |
| | | | JP 2002534657 | A | 15-10-2002 |
| | | | JP 2006208390 | A | 10-08-2006 |
| | | | JP 2006227013 | A | 31-08-2006 |
| | | | US 6592822 | B1 | 15-07-2003 |
| | | | US 2003235919 | A1 | 25-12-2003 |
| | | | WO 9958955 | A1 | 18-11-1999 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20160089967 A
- CN 105628657 A
- CN 105136763 A
- CN 103868901 A
- CN 104101588 A
- CN 105548128 A
- CN 106872424

**Non-patent literature cited in the description**

- **ALLISON SCHAAP et al.** *Optical classification of algae species with a glass lab-on-a-chip*
- **LINDSEY, R.** *What are Phytoplankton? : Feature Articles,* 16 July 2010, https://earthobservatory.nasa.gov/Features/Phytoplankton/?src.
- **BERDALET, E. ; FLEMING, L. E. ; GOWEN, R. ; DAVIDSON, K. ; HESS, P. ; BACKER, L. C. ; ENEVOLDSEN, H.** Marine harmful algal blooms, human health and wellbeing: challenges and opportunities in the 21st century. *Journal of the Marine Biological Association of the United Kingdom,* 2016, vol. 96 (1), 61-91
- **VALE, P. ; BOTELHO, M. J. ; RODRIGUES, S. M. ; GOMES, S. S. ; SAMPAYO, M. A.** Two decades of marine biotoxin monitoring in bivalves from Portugal (1986-2006): a review of exposure assessment. *Harmful Algae,* 2008, vol. 7 (1), 11-25
- **ZOHDI, E. ; ABBASPOUR, M.** Harmful algal blooms (red tide): a review of causes, impacts and approaches to monitoring and prediction. *International journal of environmental science and technology,* 2019, vol. 16 (3), 1789-1806
- Advancements and continuing challenges of emerging technologies and tools for detecting harmful algal blooms, their antecedent conditions and toxins, and applications in predictive models. **GLIBERT, P. M. ; PITCHER, G. C. ; BERNARD, S. ; LI, M.** In Global Ecology and Oceanography of Harmful Algal Blooms. Springer, 2018, 339-357
- **SHIN, Y. H. ; BARNETT, J. Z. ; GUTIERREZ-WING, M. T. ; RUSCH, K. A. ; CHOI, J. W.** A hand-held fluorescent sensor platform for selectively estimating green algae and cyanobacteria biomass. *Sensors and Actuators B: Chemical,* 2018, vol. 262, 938-946
- Photosynthetic Pigments. **SPEER, B. R.** Berkeley Museum of Paleontology. University of California, 1997

- **GONÇALVES, S.** Manual para a avaliação da qualidade biológica da água em Lagos e Albufeiras segundo a directiva quadro da agua. Instituto da Água, 2009, 1-40
- **JOHNSON, Z.I. ; MARTINY, A.C.** *Techniques for quantifying phytoplankton biodiversity,* 2015, vol. 7, 299-324
- **SU, R. ; CHEN, X. ; WU, Z. ; YAO, P. ; SHI, X.** Assessment of phytoplankton class abundance using fluorescence excitation-emission matrix by parallel factor analysis and nonnegative least squares. *Chinese journal of oceanology and limnology,* 2015, vol. 33 (4), 878-889
- **ZHANG, Q. Q. et al.** *Discrimination of phytoplankton classes using characteristic spectra of 3D fluorescence spectra,* 2006, vol. 63 (2), 361-369
- **PINTO, V. C. ; SOUSA, P. J. ; CARDOSO, V. F. ; MINAS, G.** Optimized SU-8 processing for low-cost microstructures fabrication without cleanroom facilit. *Micromachines,* 2014, vol. 5 (3), 738-755
- **HE, H. ; CHEN, F. ; LI, H. ; XIANG, W. ; LI, Y. ; JIANG, Y.** Effect of iron on growth, biochemical composition and paralytic shellfish poisoning toxins production of Alexandrium tamarense. *Harmful Algae,* 2010, vol. 9 (1), 98-104
- **ZHANG, F. ; HE, J. ; SU, R. ; WANG, X.** Assessing Phytoplankton Using a Two-Rank Database Based on Excitation-Emission Fluorescence Spectra. *Applied spectroscopy,* 2011, vol. 65 (1), 1-9
- **MAYA-HERNÁNDEZ, P. M. ; ÁLVAREZ-SIMÓN, L. C. ; SANZ-PASCUAL, M. T. ; CALVO-LOPEZ, B.** An integrated low-power lock-in amplifier and its application to gas detection. *Sensors,* 2014, vol. 14 (9), 15880-15899
- **ALLISON SCHAAP et al.** Optical classification of algae species with a glass lab-on-a-chip. *Lab on a Chip,* 27 January 2012